Europäisches Patentamt

European Patent Office

Office européen des brevets

(11) Numéro de publication: **0 378 477**

**A1**

# (12) DEMANDE DE BREVET EUROPEEN

(21) Numéro de dépôt: **90400071.8**

(51) Int. Cl.5: **A61K 37/02**

(22) Date de dépôt: **10.01.90**

(30) Priorité: **12.01.89 FR 8900325**

(43) Date de publication de la demande:
**18.07.90 Bulletin 90/29**

(84) Etats contractants désignés:
**AT BE CH DE DK ES GB GR IT LI LU NL SE**

(71) Demandeur: **FONDATION NATIONALE DE TRANSFUSION SANGUINE**
**6, rue Alexandre Cabanel**
**F-75739 Paris Cédex 15(FR)**

(72) Inventeur: **Boffa, Georges**
**62, chemin Vauhallan**
**F-91120 Palaiseau(FR)**

(74) Mandataire: **Warcoin, Jacques et al**
**Cabinet Régimbeau 26, avenue Kléber**
**F-75116 Paris(FR)**

(54) Facteur inhibiteur du complément, sa caractérisation et ses applications.

(57) La présente invention concerne une protéine FIC caractérisée en ce que :
- elle est plus particulièrement présente dans le sérum et le plasma de femmes enceintes ou de femmes soumises à une stimulation ovarienne en vue d'une fécondation artificielle réalisée in vitro ou dans les trompes utérines ;
- elle a un poids moléculaire déterminé au gel de polyacrylamide SDS de 84 000 ± 2 000 ;
- elle possède un effet inhibiteur constant et très marqué dose-dépendant sur la prolifération cellulaire.

EP 0 378 477 A1

## FACTEUR INHIBITEUR DU COMPLEMENT, SA CARACTERISATION ET SES APPLICATIONS

La présente invention a pour objet une nouvelle protéine ayant une activité inhibitrice de la lyse cellulaire complément dépendante, sa caractérisation et son application.

Cette protéine qui possède une activité inhibitrice du complément a été mise en évidence dans le sérum et le plasma de la femme gestante.

Les mécanismes naturels de défense immunologique sont essentiels pour le maintien de l'équilibre physiologique de l'individu, mais représentent un facteur limitant de survie lorsqu'ils sont mis en jeu lors des hétérogreffes d'organes et au cours des mécanismes autoimmuns. Dans ces situations, ces mécanismes doivent être maîtrisés, c'est le rôle des agents thérapeutiques tels que les glucocorticoïdes et la cyclosporine A. Les mécanismes de défense immunitaire naturels paraissent localement abolis lors de la nidation de l'oeuf fécondé et pendant tout le cours de la gestation pour permettre le maintien et le développement du foetus.

Pendant la gestation, on a décrit chez la femme des modifications quantitatives et qualitatives des alpha-2-macroglobulines dans le sérum ou le plasma.

Deux d'entre elles ont été identifiées :

. la "Pregnancy Zone Protein" (PZP)

. la "Pregnancy Associated Plasma Protein A" (PAPP-A).

Leur description structurale a montré qu'elles sont formées de sous-unités, chacune d'entre elles est composée par deux chaînes polypeptidiques liées par liaisons covalentes, ce qui a permis de les apparenter à l'alpha-2-macroglobuline du sérum et du plasma humain normal. Ces molécules possèdent des caractéristiques immunochimiques différentes de l'alpha-2-macroglobuline, ce qui permet de les identifier et d'observer leur présence avec l'alpha-2-macroglobuline commune pendant la durée de la gestation. Le rôle respectif de ces molécules au cours de la gestation n'a pas été démontré.

. La PAPP-A inhibe in vitro l'activité hémolytique dépendante du complément (Bischof 1979, 81,84).

.La PZP inhibe la transformation blastique provoquée au cours de la réaction lymphocytaire mixte (Dember 1975, Stimson 1976-80, Horne 1979).

Plus récemment, il a été montré qu'il existe dans le sérum de femmes au cours du troisième trimestre de gestation une alpha-2-macro-globuline qui possède une activité inhibitrice du complément associée à un effet anticytotoxique sur les cellules T stimulées. Son effet, comme celui de la cyclosporine, paraît s'exprimer par l'inhibition de la stimulation allogénique des lymphocytes T. Elle porte le nom de Protéine Inhibitrice du Rejet de Greffe (IRG).

La présente invention concerne une protéine dénommée Facteur Inhibiteur du Complément (ou FIC) caractérisée en ce que, outre son activité d'inhibition du complément :

- elle est présente dans le sérum et le plasma de femmes enceintes ou de femmes soumises à une stimulation ovarienne en vue d'une fécondation artificielle réalisée in vitro ou dans les trompes utérines ;
- elle est présente, avec une plus faible teneur, dans le sérum et/ou le plasma normal ;
- elle migre souvent, portée par une alpha-2-globuline en électrophorèse sur acétate de cellulose ;
- elle a un poids moléculaire déterminé au gel de polyacrylamide SDS de 84 000 ± 2 000 ;
- elle possède un effet inhibiteur constant et très marqué dose-dépendant sur la prolifération cellulaire.

Le complément est un facteur présent dans le sérum sanguin qui se fixe sur le complexe antigène-anticorps et renforce l'action destructrice de l'anticorps.

L'activité du complément est, en général, mesurée en évaluant l'activité de l'échantillon à doser en dilution limite, à lyser des globules rouges de moutons sensibilisés par des anticorps de lapins anti-globules de moutons.

La protéine en cause, FIC, est capable d'inhiber le complément et cette activité peut être mesurée dans un test adapté du test précédent.

Cette protéine, le FIC, n'est souvent pas décelable dans le sérum ou le plasma humain total de sujets hommes ou femmes hors gestation, mais après purification, elle peut être détectée mais avec une faible teneur.

Par contre, le FIC est détectable dans le sérum total des femmes en cours de troisième trimestre de gestation dans un tiers des cas et après purification dans la quasi totalité des cas. Il en va de même dans le cas de femmes soumises à une stimulation ovarienne.

Comme cela a été dit, il s'agit d'une alpha-2-globuline en électrophorèse sur cellogel et son comportement en "chromatofocusing polybuffer exchanger" indique un point isoélectrique compris entre 3,9 et 4,6. Elle est thermolabile et son activité est détruite par les réducteurs. Son activité n'est pas modifiée par le SDS.

Après électrophorèse préparative en gel de polyacrylamide SDS, l'activité se situe, par rapport aux marqueurs de poids moléculaire, sur une zone correspondant à 84 000 daltons.

L'activité du FIC dans les différents lots purifiés est supérieure à 200 000 unités par unité d'absorption (UA) à 280 nm.

Le FIC n'est pas révélé par les immunsérums monospécifiques suivants :
- anti-alpha-2-macroglobuline plasmatique,
- anti-PZP,
- anti-PAPP-A.

Les immun-sérums anti-FIC mettent en évidence un complexe antigène anticorps précipitant. Ce complexe en Ouchterlony s'individualise de celui formé par le complexe [anti-PZP - PZP].

Cependant, une réaction d'identité immunologique partielle avec la PZP n'est pas à exclure.

L'invention a également pour objet un procédé d'obtention de la protéine FIC dans lequel :
. on fractionne les sérums présentant une activité inhibitrice du complément, éventuellement additionnés d'inhibiteurs des endoprotéases, de façon à isoler les fractions migrant avec les alpha-2-macroglobulines pour obtenir des fractions riches en FIC,
. le FIC est éventuellement purifié de ces fractions à travers au moins une étape de chromatographie en suivant la présence du FIC par mesure de son activité inhibitrice du complément.

Bien que les sérums de femmes enceintes soient plus riches en FIC que les sérums normaux, ceux-ci peuvent également être utilisés pour préparer du FIC, comme cela sera décrit dans les exemples, en utilisant différentes méthodes de fractionnement.

Le fractionnement et la purification peuvent être réalisés par de nombreuses méthodes qui sont connues dans le domaine du fractionnement des protéines du sérum comme par exemple la filtration sur gel, l'ultracentrifugation, le relargage par les sels neutres, la précipitation fractionnée à l'aide d'un agent précipitant (tel qu'un alcool, un polyalkylène-glycol, ou le Rivanol), la chromatographie d'adsorption, la chromatographie sur résine échangeuse d'ions ou la chromatographie d'affinité, ou bien une combinaison de ces technologies.

Le fractionnement et la purification plus particulièrement utilisés font appel aux étapes suivantes, représentées sur le schéma ci-après.

EXEMPLE DE PREPARATION :

Mélange sérums femmes gestantes

+ Inhibiteurs

Lysine sépharose ⟶ Plasmine - Plg

Précipitation euglobulines ⟶ Alpha 2 M

AcA 22

DEAE Sephacel

CM Affigel "blue"

Affigel 10 Ig anti SHN

FIC

Le FIC présent dans le sérum en présence des antiprotéases "Soybean Trypsin Inhibitor" (STI) et "Phenyl Methane Sulfonyl Fluoride" (PMSF) est épuré des contaminations résultant de l'activation du plasminogène en plasmine sur lysine sépharose et recueillie dans la fraction non adsorbée.

Cette fraction est ensuite soumise :

- à une étape de précipitation en solution saline acide à faible molarité,

- puis à une filtration sur Ultrogel AcA 22, en particulier en tampon phosphate de Na 0,05, NaCl 0,25 M, pH 7,8,

- une chromatographie sur DEAE sephacel, en particulier en tampon d'équilibre tris 10 mM, NaCl 20 mM, HCl, pH 7,

- une chromatographie d'affinité sur CM Affigel blue, en particulier en tampon d'équilibre tris 0,02 M, NaCl 0,5 M, pH 7,5.

La purification est obtenue dans un gradient de molarité de NaCl compris entre 0,5 M et 1,5 M.

Il est possible d'améliorer la purification en effectuant une immunopurification sur Ig, dirigées contre des contaminants, fixées sur support solide.

Enfin, dans le cas présent, on donne dans les exemples, le comportement du FIC sur de nombreux supports qui permettent de purifier ce produit selon d'autres variantes grâce à de nombreuses combinaisons des techniques mentionnées.

Le produit en cause peut également être obtenu à partir de cultures cellulaires transformées ou non qui produisent ce facteur FIC.

Le FIC est également présent en quantités très importantes dans les fractions I, II, III et I, III du fractionnement de Cohn.

Le FIC selon l'invention peut être utilisé dans tous les cas où une inhibition du complément est

4

recherchée, tant in vivo qu'in vitro.

In vivo, le FIC est utilisable à titre de médicament, en particulier dans les pathologies comportant une augmentation des taux sériques de complément pouvant bénéficier, dans certains cas, de l'apport de FIC, en particulier dans le traitement des états inflammatoires et des chocs septiques.

Le FIC peut également être combiné avec une autre protéine, notamment une alpha-2-macroglobuline.

Enfin, la présente invention concerne les anticorps monoclonaux dirigés contre la protéine FIC.

Les caractéristiques et avantages de la présente invention seront mieux compris à la lecture des exemples ci-après.

## Exemple 1

Le FIC est isolé à partir d'un mélange de sérums de femmes gestantes en utilisant le schéma de séparation mentionné précédemment. Les conditions d'utilisations sont décrites à l'exemple 2.

On part d'un mélange de 100 ml de sérum qui est additionné d'inhibiteurs polyvalents des endoprotéases, parmi polybrène, aprotinine, STI, PMSF, et préalablement traités sur une résine lysine sépharose afin d'éliminer les contaminations de plasmine.

L'éluat est dialysé contre un tampon acétate pH 5,5 et le culot centrifugé est repris dans un tampon.

Le culot repris est filtré sur Ultrogel AcA 22 et on récupère les fractions activées.

Le filtrat est dialysé puis chromatographié sur résine DEAE.

L'éluat est purifié sur résine CM Affigel blue par chromatographie d'affinité.

## Exemple 2

## Comportement sur les supports chromatographiques (à 4°C)

### En DEAE Séphacel

En tampon d'équilibre tris 10 mM, NaCl 20 mM, HCl pH 7,0, l'activité du sérum total de femme gestante est retrouvée dans les fractions éluées par un gradient continu de concentration de NaCl dans une zone de molarité comprise entre 120 et 250 mM. La même zone d'élution du FIC est observée avec une préparation d'euglobulines de sérum de femme gestante du troisième trimestre ou de fraction macromolécule obtenue par chromatographie d'exclusion sur Ultrogel AcA 22 en tampon Tris 0,01 M, NaCl 0,5 M, pH 7,8.

### En chromatographie HPLC TSK 545 DEAE

Le FIC, présent avec les alpha-2-macroglobulines issues d'une préparation d'Ultrogel AcA 22, s'élue comme précédemment avec les protéines à fortes charges électronégatives.

### En "chromatofocusing polybuffer exchanger"

En tampon imidazol 25 mM, HCl, pH 7,4, l'élution du FIC est obtenue dans une zone de pH comprise entre 4,6 et 3,9.

### En Ultrogel AcA 22

En tampon phosphate de sodium 0,05 M, NaCl 0,25 M, pH 7,8, le FIC est détecté dans la fraction macromoléculaire et dans une zone d'élution des protéines de poids moléculaire plus faible que celui des macromolécules. La répartition de l'activité inhibitrice du complément varie d'un lot à l'autre.

En HPLC TSK G 4000 SW

En tampon tris 0,01 M, NaCl 0,5 M, pH 7,0, une élution en 100 minutes est obtenue, le FIC est détecté sur les mêmes zones d'élution que pour l'Ultrogel AcA 22.

En HPLC Superose 12

Les performances de ce support sont comparables à celle du TSK G 4000 SW pour la purification du FIC sérique.

En Sépharose 6B Chélate Zn

En tampon 0,5 M NaCl, Tris 0,02 M, acide acétique pH 7,3, le FIC présent dans le sérum de femme gestante est élué dans une zone de gradient de pH comprise entre 6,0 et 5,0. Dans cette zone deux pics s'individualisent, le premier comprend 29 % et le second 70 % de l'AIC recueillie.

En octyl-sépharose

En tampon d'équilibre NaCl 4 M, tris 0,01 M, pH 7,1, le FIC sérique est fortement adsorbé sur le support et la diminution de molarité du NaCl est sans effet pour la désorption. Le chlorhydrate de guanidine à 6 M, pH 8,0, permet l'élution. Le rendement en activité est de 30 %.

En phényl-sépharose

Dans des conditions comparables à l'octyl-sépharose, le FIC donne les mêmes résultats avec un faible rendement.

En HA Ultrogel

En tampon d'équilibre phosphate de Na 5 mM, pH 6,8, le FIC possède une forte affinité pour l'hydroxyapatite et son élution s'obtient par un tampon phosphate Na concentré à 0,2 M, pH 6,8.

En CM Affigel blue

En tampon d'équilibre tris 0,02 M, NaCl 0,15 M, pH 7,25, la purification du FIC issu, soit du sérum total, soit des euglobulines, soit d'une fraction d'Ultrogel AcA 22 obtenue à partir d'euglobulines, s'obtient dans un gradient de molarité de NaCl compris entre 0,5 M et 1,5 M.
Les rendements en activité sont supérieurs à 70 %.

En lysine sépharose

En tampon d'équilibre phosphate de Na 30 mM, NaCl 0,15 M, pH 7,4, le FIC présent dans le sérum en présence des antiprotéases STI et PMSF est épuré des contaminations résultant de l'activation du plasminogène en plasmine et recueilli dans la fraction non adsorbée.

**Exemple 3**

**Caractéristiques physicochimiques et biologiques de la molécule "FIC"**

Elle est présente dans le sérum et le plasma de femme gestante ou de femme soumise à une stimulation hormonale ovarienne en vue d'une fécondation artificielle réalisée in vitro (FIV) ou dans les trompes utérines.

Elle est présente en très faible quantité dans le sérum d'homme ou de femme hors gestation.

Elle migre portée par une alpha-2-globuline en électrophorèse sur acétate de cellulose.

En chromatographie en gel filtrant, en milieu salin dépourvu de détergent et à pH physiologique, la molécule s'élue avec les macroglobulines du sérum ou du plasma.

A l'électrophorèse en gel de polyacrylamide, le FIC, présent avec les alpha-2-macroglobulines purifiées, se sépare des sous-unités des alpha-2-macroglobulines (poids moléculaire : 370 000 daltons) quelle que soit la nature du tampon, en présence de SDS ou en tampon glycine dépourvu de détergent.

Le poids moléculaire du FIC déterminé en gel de polyacrylamide SDS est de 84 000 ± 2 000.

Son point isoélectrique est compris entre 4,5 et 3,9.

Cette molécule incubée en présence de complément inhibe l'hémolyse des globules rouges de moutons revêtus d'anticorps anti-mouton.

Le FIC ne possède pas à lui seul de propriété anticytotoxique comparable à celle donnée par le complexe [alpha-2-macromolécule-FIC)

Il n'a pas d'activité estérolytique sur les substrats synthétiques tels que le Tosyl Arginine Méthyl Ester (TAME), le Benzoyl Arginine Ethyl Ester (BAEE) et l'Acétyl Tyrosine Ethyl Ester (ATEE).

L'activité inhibitrice du complément (AIC) n'est pas modifiée par les détergents tels que l'urée 6 M, le chlorhydrate de guanidine 6 M, le tween à 0,02 %. Le SDS à 0,1 % est sans effet, à 1% la molécule perd 75 % de son AIC.

Les inhibiteurs des protéases suivants sont sans effet sur l'AIC du FIC : Aprotinine (Iniprol) à 2 000 U/ml, Phényl Méthane Sulfonyl Fluoride (PMSF) à 10 mM, le polybrène à 50 μg/ml, le Soybean Trypsin Inhibitor (STI) à 50 μg/ml, la benzamidine à 5 mM, l'acide aminocaproïque 0,2 M.

Seul le Diisopropyl Fluoro Phosphate (DFP) à 10 mM inhibe complètement l'AIC.

L'AIC de la molécule est abolie par les réducteurs tels que le béta-mercaptoéthanol à 1 %.

La molécule, très soluble, est précipitée en présence d'acide caprylique, le rivanol à 0,4 % et le polyéthylèneglycol (PEG) 4000 à partir de 6 %.

Elle précipite à faible molarité saline et à pH acide égal ou inférieur à 5,5, c'est une euglobuline.

Les dialyses en tampon tris 10 mM NaCl 0,5 M, pH 7,8 ou en tampon phosphate 50 mM, NaCl 0,25 M, pH 7,8, ne modifient pas l'activité de la molécule.

L'activité de la molécule n'est pas modifiée par la lyophilisation ou les filtrations stérilisantes sur membrane. La molécule est thermolabile, elle s'inactive rapidement au-dessus de 37° C.

## Exemple 4

### Evolution du facteur inhibiteur du complément

Le FIC n'est pas décelable, le plus souvent, dans le sérum ou le plasma humain total.

Sa présence est décelable seulement après purification partielle par chromatographie d'exclusion sur gel filtrant ou par précipitation avec les euglobulines.

Dans le premier cas, il est associé aux alpha-2-macroglobulines. Il est inconstamment mis en évidence dans les préparations d'alpha-2-macroglobuline issues du sérum humain normal individuel (homme ou femme).

Quand il est détectable, il s'exprime le plus souvent par une faible teneur comme en témoigne son activité inhibitrice du complément (AIC).

Par contre, la présence du FIC est révélée d'après son AIC dans le sérum entier des femmes en cours du troisième trimestre dans un tiers de cas (Tableau I).

Après purification des alpha-2-macroglobulines issues soit de sérum individuel, soit de mélange de sérums de femmes gestantes, le FIC est décelable dans tous les cas.

Après purification des euglobulines sériques, le FIC est identifié dans cette fraction dans 90 % des cas (Tableau II).

Le FIC peut également être mis en évidence au cours des fécondations in vitro (FIV) ou dans les trompes utérines (GIFT) qui se préparent par un traitement hormonal progestatif précédé par une phase d'inhibition hypophysaire.

Sous l'influence de la stimulation ovarienne l'AIC apparaît précocement dans le sérum total des femmes et se retrouve dès la première semaine, même en l'absence de gestation comme le confirment les dosages des béta-HCG. Chez un tiers des cas des sujets analysés, le FIC est détecté directement dans le sérum total (Tableau III).

Tableau I

| REPARTITION DE L'ACTIVITE INHIBITRICE DU COMPLEMENT (AIC) DANS QUATRE LOTS DE SERUMS DE FEMMES GESTANTES PRELEVES PENDANT LE TROISIEME TRIMESTRE DE GESTATION | | | |
|---|---|---|---|
| | Sérums entiers | AIC$^+$ | AIC$^-$ |
| 1ère série (1986) | 205 | 72 (35%) | 133 (65%) |
| 2ème série (1er trim. 1987) | 124 | 41 (33%) | 83 (67%) |
| 3ème série (2ème trim. 1987) | 103 | 36 (35%) | 67 (65%) |
| 4ème série (3ème trim. 1987) | 133 | 29 (22%) | 104 (78%) |
| TOTAL | 565 | 178 (32%) | 387 (68%) |
| Sérums témoins (20 F, 10 H) | 30 | 0 | 30 |

Tableau II

| REPARTITION DE L'AIC DANS LES SERUMS ENTIERS DU TROISIEME TRIMESTRE DE GESTATION ET PARMI LES EUGLOBULINES | Sérums entiers | | Euglobulines | |
|---|---|---|---|---|
| | AIC$^-$ | AIC$^+$ | AIC$^-$ | AIC$^+$ |
| Gestantes 3ème trimestre : 65 | 43 (66%) | 22 (34%) | 6 (10%) | 59 (90%) |
| Témoins : 30 | 30 (100%) | | 29 (97%) | 1 (3%) |

Tableau III

| REPARTITION DE L'AIC DANS LES SERUMS DE FEMMES SOUMISES A UNE STIMULATION OVARIENNE SUIVIE D'UNE TRANSPLANTATION D'EMBRYON (PRELEVEMENT EFFECTUE AVEC LE PREMIER DOSAGE BETA-HCG) | | | |
|---|---|---|---|
| | Sérums entiers | AIC$^+$ | AIC$^-$ |
| Stimulation ovarienne | | | |
| Femmes non gestantes | 83 | 25 (30%) | 58 (70%) |
| Femmes gestantes | 20 | 8 (40%) | 12 (60%) |
| TOTAL | 103 | 33 (32%) | 70 (68%) |

**Exemple 5**

La protéine FIC inhibitrice du complément est décelée après chromatographie sur gel filtrant de sérum humain normal dans les conditions suivantes : colonne d'Ultrogel AcA22 en tampon phosphate de Na 50 mM, NaCl 250 mM, pH 7,8. Rapport ml de gel/mg de protéines : 1/1.

Les résultats obtenus sont rassemblés dans le tableau IV :

Tableau IV

| MISE EN EVIDENCE DES ALPHA-2-MACROGLOBULINES INHIBITRICES DU COMPLEMENT DANS LE SERUM DE L'HOMME NORMAL ET DE LA FEMME ENCEINTE | | | | | |
|---|---|---|---|---|---|
| | | ACTIVITE INHIBITRICE DU COMPLEMENT | | | |
| | Volume (ml) | Sérum entier | F.I. (U/ml) | Act. totale (U) | Sérum de départ (U/ml) |
| 1. S.H.N. | 20 | 0 | 10 000 | 35 000 | 1 790 |
| 2. S.H.N. | 20 | 0 | 31 260 | 66 400 | 3 320 |
| 3. S. gestante | 20 | 0 | 90 000 | 247 500 | 12 375 |
| 4. S. gestante | 20 | 0 | 59 300 | 213 400 | 10 670 |
| 5. S. gestante | 22 | 0 | 38 400 | 96 000 | 4 360 |
| 6. S. gestante | 24 | 0 | 41 000 | 106 340 | 4 430 |
| 7. S. gestante | 24 | 0 | 56 000 | 241 000 | 10 040 |
| 8. S. gestante | 24 | 0 | 71 900 | 316 000 | 13 166 |
| F.I. : Fraction Macromoléculaire issue de l'Ultrogel AcA22 | | | | | |

Les sérums de femmes gestantes contiennent dans leur fraction alpha-2-macroglobuline de 1 000 à 14 000 Unités Inhibitrices par ml de sérum. Ce taux dans le S.H.N. est compris entre 1 500 et 3 500 U.I. par ml de sérum. On note que l'activité inhibitrice ne se révèle pas directement dans la plupart des cas dans le sérum entier.

## Exemple 6

Les alpha-2-macroglobulines sériques sont obtenues par précipitation en présence de polyéthylène glycol (PEG 4 000 ou 6 000). L'activité inhibitrice se distribue en presque totalité dans le précipité obtenu avec 6 % de PEG. Le précipité 6-16 % contient moins de 1 % de l'activité inhibitrice totale.

Des sérums individuels d'homme normal et de femme enceinte sont dilués au 1/3 en tampon $NaH_2PO_4$ 25 mM et $Na_2HPO_4$ 50 mM.

Le PEG 4 000 à 50 % est additionné sous agitation pendant 30 minutes à 4° C. Les culots obtenus par centrifugation sont repris dans le volume de départ et dialysés. Leurs activités sont mesurées vis à vis du complément (Tableau V).

L'activité inhibitrice s'est développée dans la fraction enrichie en alpha-2-macroglobuline obtenue individuellement. L'activité inhibitrice est de même intensité chez l'homme et chez la femme enceinte.

Le PEG seul à des concentrations allant de 0,1 à 6 % est sans effet sur l'activité du complément lors de l'hémolyse immune.

Tableau V

| DEVELOPPEMENT DE L'ACTIVITE INHIBITRICE DU COMPLEMENT OBTENUE PAR PRECIPITATION AU PEG DANS LE SERUM D'HOMME ET DE FEMME ENCEINTE | | |
|---|---|---|
| | Activités U/ml sérum entier | PEG 4 000 6 % culot dissous et dialysé |
| Sérum homme normal | | |
| 1. | | 186 600 |
| 2. | | 161 600 |
| 3. | | 171 000 |
| 4. | | 166 500 |
| 5. | | 126 400 |
| 6. | | 112 600 |
| 7. | 347 300 | 112 300 |
| 8. | | 104 500 |
| 9. | | 113 500 |
| Sérum femme gestante | | |
| 11. | | 170 400 |
| 12. | 116 300 | 173 900 |
| 13. | | 111 200 |
| 14. | | 63 700 |
| 15. | | 169 800 |
| 16. | | 161 600 |
| 17. | | 112 400 |
| 18. | | 199 500 |
| 19. | | 168 700 |
| 20. | | 171 400 |

## Exemple 7

Les alpha-2-macroglobulines sériques sont purifiées par précipitation en présence de rivanol suivie d'une chromatographie d'exclusion sur gel de sépharose.

Un mélange de sérum humain de 60 ml a été additionné de STI et de phényle méthane sulfone fluorure (PMSF) puis dilué en tampon $K_2HPO_4$, $KH_2PO_4$ 30 mM, pH 7,2.

Une poudre de rivanol est ajoutée sous agitation douce jusqu'à une concentration finale de 0,4 % à la température ambiante. Après centrifugation, le culot est repris en NaCl 0,83 M pour précipiter le rivanol. Un passage sur colonne de G 25 coarse élimine les contaminants résiduels de rivanol. Une colonne de sépharose CL 6B équilibrée en tampon phosphate Na/K, pH 7,2 est utilisée avec un rapport ml de gel/mg de protéine de 3/4.

Cinq fractions sont obtenues, les alpha-2-macroglobulines se distribuent dans les fractions II et III.

Le tableau VI montre que l'activité inhibitrice du complément se distribue dans les macromolécules issues soit du sérum d'homme normal, soit du sérum de femmes gestantes, avec un maximum dans la fraction III.

EP 0 378 477 A1

Tableau VI

| REPARTITION DE L'ACTIVITE INHIBITRICE DU COMPLEMENT DANS LES FRACTIONS DE SEPHAROSE | | | | | |
|---|---|---|---|---|---|
| | I | II | III | IV | V |
| **S.H.N. :** | | | | | |
| A.I.C./Unités | 79 230 | 29 120 | 177 920 | 0 | 0 |
| **S. GESTANTE :** | | | | | |
| A.I.C./Unités | 34 200 | 5 890 | 54 600 | 0 | 0 |
| S.H.N. : sérum d'homme normal  S. GESTANTE : sérum de femme enceinte | | | | | |

**Exemple 8**

Les alpha-2-macroglobulines sont décelées en abondance dans des fractions obtenues par précipitation alcoolique selon la filière de Cohn.

Les activités inhibitrices du complément sont recherchées à 5 niveaux :

. le plasma de départ,

. le sérum issu de ce plasma par recalcification et addition de thromboplastine,

. la fraction I, II, III,

. la fraction IV,

. la fraction I, III.

Chaque échantillon est analysé avant dialyse, puis après diayle et dans ce cas avec ou sans inhibiteurs.

Une activité inhibitrice du complément est décelée le long de la filière de fractionnement au niveau de la fraction I, II, III et en aval dans la fraction I, III.

Le sérum issu par recalcification du plasma, les fractions I, II, III et I, III sont traitées par filtration sur AcA22 en tampon phosphate de sodium 50 mM, NaCl 250 mM, pH 7,8 à 4° C sur une colonne 16 mm de diamètre contenant 220 ml de gel avec un débit de 5 ml/heure.

Des fractions de 7 ml sont recueillies et dosées en AIc.

Le sérum révèle une seule zone d'élution du facteur actif au niveau alpha-2-macroglobulines.

Les fractions I, II, III donnent deux zones dont l'une se superpose à la fraction alpha-2-M, l'autre de plus faible taille moléculaire correspondant à la sous-unité de l'alpha-2-M. La fraction I et III donnent une seule zone d'élution au niveau de l'alpha-2-M avec un rendement d'environ 12 % par rapport à la fraction I,III de départ.

**Revendications**

1) Protéine FIC caractérisée en ce que :

- elle est présente dans le sérum et le plasma de femmes enceintes ou de femmes soumises à une stimulation ovarienne en vue d'une fécondation artificielle réalisée in vitro ou dans les trompes utérines ;

- elle est présente avec une plus faible teneur dans le sérum et/ou le plasma normal ;

- elle a un poids moléculaire déterminé au gel de polyacrylamide SDS de 84 000 ± 2 000 ;

- elle possède un effet inhibiteur constant et très marqué dose-dépendant sur la prolifération cellulaire.

2) Protéine FIC selon la revendication 1, caractérisée en ce que :

- elle a un point isoélectrique compris entre 3,9 et 4,6,

- elle n'est pas révélée par les immunsérums monospécifiques

. anti-alpha-2-macroglobulines plasmatiques,

11

. anti-PZP

. anti-PAPP-A.

3) Procédé de préparation de la protéine FIC selon l'une des revendications 1 et 2, caractérisé en ce que :

- on fractionne les sérums présentant une activité inhibitrice du complément, éventuellement additionnés d'inhibiteurs des endoprotéases de façon à isoler les fractions migrant avec les alpha-2-macroglobulines pour obtenir des fractions riches en FIC,

- le FIC est éventuellement purifié de ces fractions à travers au moins une étape de chromatographie en suivant la présence du FIC par mesure de son activité inhibitrice du complément.

4) Procédé selon la revendication 3, caractérisé en ce que la protéine FIC est obtenue à partir du schéma d'isolement ci-après :

Mélange sérums femmes gestantes

+ Inhibiteurs

↓

Lysine sépharose ⟶ Plasmine - Plg

↓

Précipitation euglobulines ⟶ Alpha 2 M

↓

AcA 22

↓

DEAE Sephacel

↓

CM Affigel "blue"

↓

Affigel 10 Ig anti SHN

↓

FIC

5) Procédé selon la revendication 3, caractérisé en ce que pour la préparation d'une fraction riche en FIC on sépare le FIC du sérum humain normal par chromatographie sur gel filtrant.

6) Procédé selon la revendication 5, caractérisé en ce que le gel filtrant est un Ultrogel AcA22.

7) Procédé selon la revendication 3, caractérisé en ce que pour la préparation d'une fraction riche en FIC on précipite la fraction riche en FIC des alpha-2-macroglobulines sériques avec environ 1 à 6 % de PEG 2 000 - 8 000.

8) Procédé selon la revendication 3, caractérisé en ce que pour la préparation d'une fraction riche en FIC on ajoute au sérum du rivanol et, à partir du précipité obtenu, on sépare le FIC par chromatographie.

9) Procédé selon la revendication 7, caractérisé en ce que la chromatographie est effectuée sur résine Sépharose CL 6B.

10) Procédé selon la revendication 3 de préparation d'une fraction riche en FIC caractérisé en ce qu'il s'agit des fractions I, II, III et des fractions I, III du fractionnement de Cohn.

11) Anticorps monoclonal dirigé contre la protéine FIC selon l'une des revendications 1 et 2.

12) A titre de médicament, la protéine FIC selon l'une des revendications 1 et 2.

13) A titre de médicament, la protéine FIC en combinaison avec une autre protéine.

14) A titre de médicament, la protéine FIC en combinaison avec une alpha-2-macroglobuline.

15) Composition pharmaceutique, caractérisée en ce qu'elle comporte à titre de principe actif au moins un médicament selon l'une des revendications 12 à 14.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 588 558  (CENTRE NATIONAL DE LA RECHERCHE SCIENTIFIQUE) <br> * En entier * <br> --- | 1-15 | A 61 K  37/02 |
| A | CHEMICAL ABSTRACTS, vol. 102, no. 1, 7 janvier 1985, page 274, résumé no. 2867m, Columbus, Ohio, US; M. ITO et al.: "The purifiction of pregnancy specific betal-glycoprotein (SP1) from third trimester serum and the immunological identification of gamma-component of SP1", & NIPPON SANKA FUJINKA GAKKAI ZASSHI 1984, 36(10), 1913-20 <br> * Résumé * <br> --- | 1-15 | |
| A | CHEMICAL ABSTRACTS, vol. 87, no. 25, 19 décembre 1977, page 510, résumé no. 198826m, Columbus, Ohio, US; G.N. THAN et al.: "Biochemical and clinico-pathological aspects of pregnancy-associated alpha-2 glycoprotein", & PROTIDES BIOL. FLUIDS 1976, 24, 223-6 <br> * Résumé * <br> --- | 1-15 | DOMAINES TECHNIQUES RECHERCHES (Int. Cl.5) <br><br> A 61 K |
| A | CHEMICAL ABSTRACTS, vol. 105, no. 1, 7 juillet 1986, page 411, résumé no. 4235q, Columbus, Ohio, US; W.C. CHOI et al.: "Protein patterns of blood plasma in pregnant women by SDS/polyacrylamide gel eletrophoresis", & TONGMUL HAKHOECHI 1985, 28(4), 237-44 <br> * Résumé * <br> ---                              -/- | 1-15 | |

Le présent rapport a été établi pour toutes les revendications

| Lieu de la recherche | Date d'achèvement de la recherche | Examinateur |
|---|---|---|
| LA HAYE | 17-04-1990 | FERNANDEZ Y BRANAS F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

    ......................................................................

& : membre de la même famille, document correspondant

EPO FORM 1503 03.82 (P0402)

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 90, no. 1, 1 janvier 1979, page 256, résumé no. 2567x, Columbus, Ohio, US; J. FOLKERSEN et al.: "Affinity chromatographic purification of the pregnancy zone protein", & J. IMMUNOL. METHODS 1978, 23(1-2), 117-25 <br> * Résumé * <br> --- | 1-15 | |
| A | PLACENTA, vol. 2, 1981, pages 29-34, W.B. Saunders Co., Ltd; P. BISCHOF: "Pregnancy-associated plasma protein-A: an inhibitor of the complement system" <br> * En entier * <br> --- | 1-15 | |
| A | US-A-4 191 533  (H. BOHN) <br> * En entier * <br> ----- | 1-15 | |

DOMAINES TECHNIQUES
RECHERCHES (Int. Cl.5)

Le présent rapport a été établi pour toutes les revendications

| Lieu de la recherche | Date d'achèvement de la recherche | Examinateur |
|---|---|---|
| LA HAYE | 17-04-1990 | FERNANDEZ Y BRANAS F.J. |